# EUROPÄISCHE PATENTSCHRIFT

(11) **EP 0 965 307 B1**
(45) Veröffentlichungstag und Bekanntmachung des Hinweises auf die Patenterteilung: **20.09.2006**
(21) Anmeldenummer: 98111300.4
(22) Anmeldetag: 19.06.1998
(51) Int. Cl.: A61B 17/16

(54) **Zum einmaligen Gebrauch bestimmte Raspel, insbesondere Knochenraspel, sowie Verfahren zu deren Herstellung**
Disposable bone rasp and related method of manufacture
Râpe à os jetable, et procédé pour sa fabrication

(43) Veröffentlichungstag der Anmeldung: 22.12.1999
(73) Patentinhaber: IMT INTEGRAL MEDIZINTECHNIK AG, 6373 Ennetbürgen (CH)
(72) Erfinder: Grünig, Daniel, 6102 Malters (CH); Geisser, Albert, 6373 Ennetbürgen (CH)
(74) Vertreter: Blum, Rudolf Emil

(56) Entgegenhaltungen:
- EP-A- 0 331 626
- EP-A- 0 472 132
- DE-C- 3 801 678
- US-A- 4 466 429

## Beschreibung

Die Erfindung betrifft eine Raspel gemäss Oberbegriff des Anspruchs 1. Ferner betrifft die Erfindung ein Verfahren zur Herstellung einer Raspel gemäss Oberbegriff des Anspruchs 9.

Raspeln, welche zur Bearbeitung von Knochenmaterial geeignet sind, und z.B. bei der Implantierung von Hüftgelenksprothesen zum Einsatz kommen, sind in verschiedenen Ausführungen bekannt. Aus DE-A-39 07 256 ist eine Knochenraspel aus Metall bekannt, die im Inneren einen Kanal zur Aufnahme des abgeraspelten Materials aufweist. Eine Raspel mit Hohlraum ist ebenfalls aus EP-A-0 331 626 bekannt. Diese Raspeln sind konventionell aus Stahl gefertigt und für den mehrmaligen Gebrauch vorgesehen, was eine entsprechende Reinigung bedingt. Diese ist arbeitsaufwendig und führt trotzdem nicht zur vollständigen Entfernung von Ablagerungen. Auch die nachfolgende Sterilisation der Raspel führt nicht zur Entfernung solcher Ablagerungen und es ist sogar möglich, dass infektiöses Material, insbesondere infektiöses Eiweiss, auch nach dem Sterilisationsvorgang ein infektiöses Potential behalten kann. Es sind daher bereits Wegwerfraspeln für den nur einmaligen Gebrauch vorgeschlagen worden. EP-A-0 563 585 und EP-A-0 574 701 zeigen Wegwerfraspeln aus Kunststoff. Bei der Herstellung solcher Raspeln aus biokompatiblem Kunststoffmaterial können sich aber Probleme mit der Raspelwirkung ergeben, insbesondere in hartem Knochenmaterial und bei längerer Anwendungsdauer. US-A-5 100 267 zeigt eine Wegwerfraspel in Halbkugelform, bei welcher die Halbkugel in normaler Weise als eigenstabiles Teil ausgeführt ist und an diesem eine scheibenförmige Basis aus Kunststoff befestigt ist, an welcher der Adapterteil zur Einspannung der Raspel in einem Antriebswerkzeug angeordnet ist. Die eigenstabile Ausführung des metallenen Raspelteiles führt zur Verwendung relativ dicken chirurgischen Stahles von mehr als 1 mm Dicke, was einen hohen Aufwand für eine Wegwerfraspel darstellt. Bei statisch ungünstigeren Formen als der Halbkugel wären nach diesem Prinzip hergestellte Raspeln annähernd gleich aufwendig, wie die normalen Raspeln zur Mehrfachverwendung.

Der Erfindung liegt daher die Aufgabe zugrunde, eine Raspel zu schaffen, welche die genannten Nachteile nicht aufweist. Insbesondere soll eine Wegwerfraspel für Knochenmaterial geschaffen werden, welche die genannten Nachteile nicht aufweist.

Dies wird bei einer Raspel der eingangs genannten Art durch die kennzeichnenden Merkmale des Anspruchs 1 erreicht.

Dadurch, dass ein innerer Stützkörper vorgesehen ist, der der Form der Raspel entspricht, kann die äussere Umhüllung aus Metall, die die Raspelzähne bildet, im Vergleich mit konventionellen Raspeln sehr dünnwandig sein. Dies senkt den Materialaufwand und kann auch den Herstellungsaufwand senken. Ferner können auf diese Weise die Raspeln auch mit dünnwandigen, eine besonders gute Schneidleistung ergebenden, Raspelzähnen versehen sein.

Bevorzugterweise ist der Stützkörper mit Rippen versehen, welche die Auflageflächen für die Umhüllung bilden und zwischen sich Hohlräume bilden, in welche das von der Raspel entfernte Knochenmaterial durch die Öffnungen bei den Raspelzähnen eintreten kann.

Der Erfindung liegt ferner die Aufgabe zugrunde, ein Verfahren zur Herstellung einer Raspel zu schaffen, welches die einfache und kostengünstige Herstellung erlaubt.

Dies wird bei einem Verfahren der eingangs genannten Art mit den kennzeichnenden Merkmalen des Anspruchs 9 erreicht.

Im folgenden werden Ausführungsbeispiele der Erfindung anhand der Zeichnungen näher erläutert. Dabei zeigt
Figur 1 eine perspektivische Ansicht einer ersten Ausführungsform der Raspel;
Figur 2 eine perspektivische Ansicht des Stützkörpers;
Figur 3 eine perspektivische Ansicht der Umhüllung der Raspel; und
Figur 4 einen Vertikalschnitt durch eine weitere Ausführungsform der Raspel.

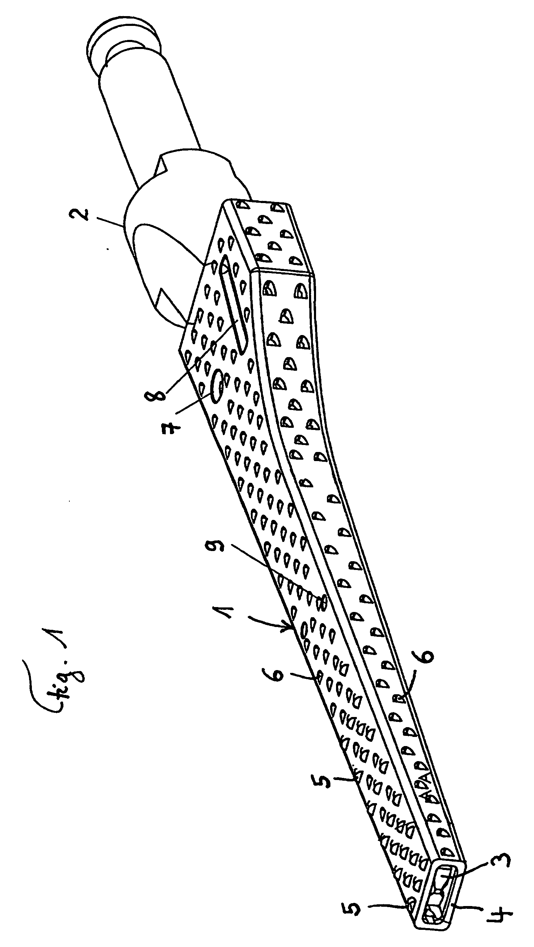

Figur 1 zeigt eine perspektivische Ansicht einer Knochenraspel 1 mit weggeschnittener Spitze, damit ein Einblick in das Innere gegeben ist. An der Knochenraspel 1 ist ein Adapter 2 befestigt, mittels welchem die Knochenraspel an ein Antriebswerkzeug angekoppelt werden kann, welches die Knochenraspel auf bekannte Weise in eine oszillierende Bewegung versetzen kann. Die Knochenraspel ist gemäss der Erfindung mit einem inneren Stützkörper 3 und einer äusseren Umhüllung 4 gebildet. Die äussere Umhüllung besteht aus Metall und weist die Raspelzähne 5 auf, von denen nur ein Teil als erhabene Raspelzähne gezeigt sind und die weiteren Zähne 6 aus zeichnerischen Gründen nur als Vertiefungen angedeutet sind. Die Raspel 1 weist eine an sich bekannte Formgebung mit einem spitz oder stumpf zulaufenden vorderen Ende und einem sich nach hinten aufweitenden hinteren Ende auf. Im gezeigten Beispiel sind zur Befestigung der Umhüllung 4 am Innenkörper Ausnehmungen vorgesehen, in welche das Metallmaterial der Umhüllung 4 eingepresst werden kann, um eine Verbindung der beiden Teile zu erzielen. In der Figur 1 ist diese Befestigungsweise durch Vertiefungen 7, 8 und 9 dargestellt.

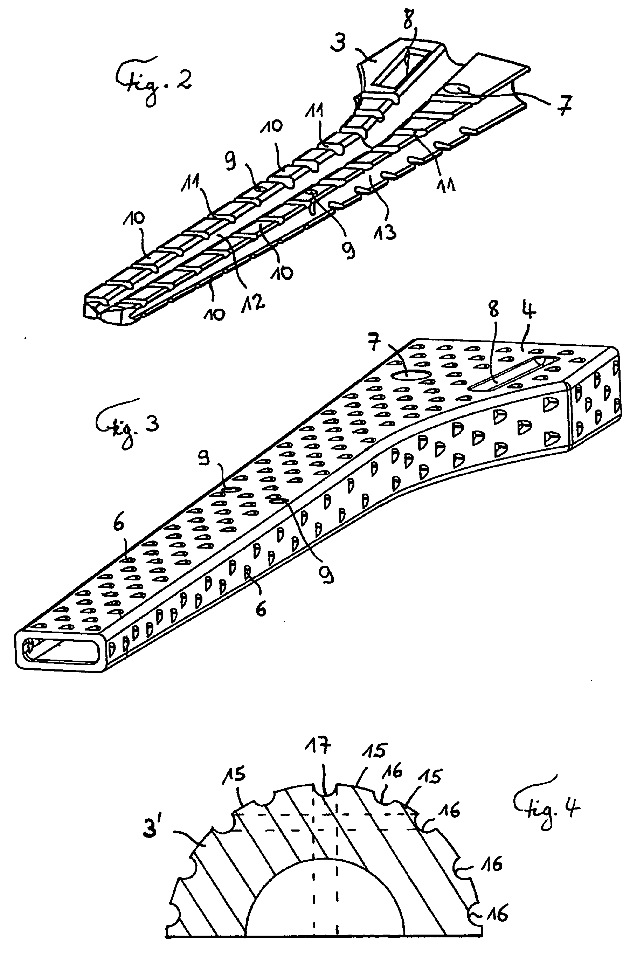

Figur 2 zeigt den Stützkörper 3 der Raspel, ebenfalls in perspektivischer Darstellung, wobei der Stützkörper gegenüber der Lage von Figur 1 um 180° um seine Längsachse gedreht ist. Der Stützkörper 3 weist im wesentlichen die Form der Raspel 1 auf, wobei sich der Stützkörper im vorliegenden Beispiel nicht bis zur Spitze der Raspel 1 hin erstreckt, sondern vor der Spitze endet. In diesem Beispiel wird also der vorderste Teil der Raspel nur von der Umhüllung alleine gebildet. Der Stützkörper bildet eine Auflage für die Umhüllung und kann vollflächig der inneren Wandung der Umhüllung angepasst sein. Bevorzugt ist allerdings, dass der Stützkörper, wie in Figur 2 gezeigt, eine Vielzahl von Stützelementen 10 aufweist, welche z.B. als Rippen bezeichnet werden können, und welche durch Ausnehmungen 11, 12 und 13 voneinander getrennt sind. Im gezeigten Beispiel bilden die Ausnehmungen 12 und 13 in der Längsrichtung der Raspel verlaufende Kanäle, während die Ausnehmungen 11 zwischen den Rippen 10 quer liegende Kanäle bilden, welche die Längskanäle 12 und 13 miteinander verbinden. Die Kanäle 11-13 erlauben es, dass das durch die Raspel abgetragene Material durch die Öffnungen der Raspelzähne in das Innere der Raspel eintritt und sich dort in den Kanälen sammelt. In der Regel genügen die Volumen der Kanäle, um alles während des Raspelvorgangs anfallende Material aufzunehmen. Es kann indes auch eine Absaugung des Materials aus den Kanälen vorgesehen sein, welche indes hier nicht dargestellt wird.

Figur 3 zeigt die Umhüllung, welche um den Stützkörper 3 von Figur 2 herum angeordnet ist und mit diesem zusammen die Raspel bildet. Auch in Figur 3 ist die Umhüllung ohne die Spitze der Raspel gezeichnet. Die Form der nicht dargestellten Spitze kann dabei den bekannten Raspelformenspitzen entsprechen und wird deshalb hier nicht weiter dargestellt.

Für das Material des inneren Stützkörpers 2 kommen insbesondere Kunststoffe oder auch Aluminium in Frage. Insbesondere sind eine Reihe von biokompatiblen Kunststoffen bekannt, welche für chirurgische Elemente eingesetzt werden, die auch den Innenkörper 3 der Raspel bilden können. Das Element 3 wird in der Regel durch ein Spritzgussverfahren hergestellt. Die Umhüllung 4 kann z.B. durch ein Tiefziehen eines Metallblechs in Längsrichtung der Raspel gebildet werden. Dabei können mehrere Tiefziehschritte notwendig werden. Es ergibt sich dann eine einstückige Umhüllung, wie sie in Figur 3 dargestellt ist. In diese Umhüllung werden die Raspelzähne durch Einstechen und Herausziehen entsprechender Materialabschnitte eingeformt, wie dies grundsätzlich bekannt ist. Danach können die Umhüllung 4 und der Stützkörper 3 miteinander verbunden werden, indem die Umhüllung über den Stützkörper geschoben wird und diese beiden Elemente miteinander verbunden werden, z.B. durch Einpressen metallener Hüllenteile in die Ausformungen 7, 8 und 9 des Stützkörpers, wie dies in Figur 1 dargestellt ist. Auch andere Befestigungsmöglichkeiten, durch Kleben oder mittels Schrauben oder Nieten, sind natürlich durchaus möglich.

Die Umhüllung kann ferner auch mehrteilig gebildet werden, z.B. durch Tiefziehen von zwei wannenartigen Hälften, welche nachher miteinander verbunden werden. Bei einer solchen Tiefzieh- bzw. Stanzoperation können auch die Raspelzähne gebildet werden. Das Verbinden der beiden Teile der Umhüllung kann durch Falzen, Schweissen, Schrauben oder durch Befestigungsmittel am Stützkörper 3 erfolgen, so dass der Stützkörper 3 die beiden Hälften der Umhüllung 4 fixiert und dadurch die Umhüllung zusammenhält. Entsprechende Nuten zur Aufnahme von Flanschen oder Falzen der Umhüllungsteile können im Stützkörper 3 vorgesehen sein.

Das Material der Umhüllung wird von üblichem chirurgischen Stahl oder einem anderen Metall gebildet. Da die Umhüllung gemäss der Erfindung die Raspel zusammen mit dem Stützkörper 3 bildet, besteht die Möglichkeit, die Umhüllung so dünn auszuführen, wie dies bei einer herkömmlichen Raspel ohne Stützkörper nicht möglich ist. So kann z.B. das Material der Umhüllung aus einem Blech von nur 0,2 mm bis 0,8 mm Dicke bestehen. Das Blech kann indes auch eine Dicke von bis zu 1 mm aufweisen oder sofern dies gewünscht ist, auch in herkömmlicher Weise eine Dicke von z.B. bis zu 4 mm. Für eine Wegwerfraspel ist es indes bevorzugt, den Materialaufwand für die Umhüllung 4 möglichst gering zu halten, was dank des Stützkörpers, der aus kostengünstigem Kunststoff hergestellt werden kann, durchaus möglich ist. Ebenso kann durch das Vorsehen sehr dünnen Metallbleches für die Umhüllung das Einbringen der Raspelzähne erleichtert werden und diese können besonders scharfkantig und damit einen guten Schnitt ergebend ausgeführt werden.

Die Form der Raspelzähne kann dabei weitgehend beliebig gewählt werden. Es können viereckig, dreieckig oder rund ausgebildete Raspelzähne vorgesehen sein. Ferner auch oval geformte Raspelzähne sowie Raspelzähne, die eine oder mehrere Einbuchtungen oder Ausbuchtungen entlang ihres äusseren schneidenden Randes aufweist.

Die Dimensionierung und die Formgebung des Stützkörpers kann ebenso in weiten Grenzen frei gewählt werden, wobei grundsätzlich gilt, dass je dünner das Material der Umhüllung 4 gewählt ist, je weniger Eigenstabilität also die Umhüllung aufweist, desto mehr Stützstellen für die Umhüllung durch den Stützkörper bereitgestellt werden müssen.

Figur 4 zeigt eine Schnittdarstellung durch einen Stützkörper 3', welcher im wesentlichen halbkugelförmig aufgebaut ist. Dabei sind ebenfalls Rippen 15 vorgesehen, welche die Auflagefläche für die nicht dargestellte, ebenfalls halbkugelförmig und an den Stützkörper angepasste Umhüllung aus Metall mit den Raspelzähnen bilden. Die Rippen sind durch Kanäle 16 voneinander getrennt. Neben den in der Figur 4 horizontal verlaufenden Kanälen 16, von denen einer mit unterbrochenen Linien dargestellt ist, können auch vertikal verlaufende Kanäle 17 vorgesehen sein, von denen ebenfalls ein Kanal mit durchbrochenen Linien angedeutet ist. Der Stützkörper 3' weist ebenfalls, wie der Stützkörper 3, einen nicht dargestellten Anschluss für den Adapter 2 auf. Der Adapter 2 kann am Innenkörper z.B. verschraubt oder durch einen Steckanschluss mit diesem verbunden sein. Der Adapter wird in der Regel nicht als Wegwerfteil ausgeführt sind und wird nach der Verwendung der Raspel 1 von dieser gelöst.

Nach der Verwendung der Raspel kann diese weggeworfen werden. Es ist möglich, die Raspel durch Zerkleinerung in ihre Grundmaterialien, z.B. Kunststoff und Metall oder Aluminium und Stahl aufzutrennen und diese Grundmaterialien je nachdem einer Wiederverwertung bzw. Verbrennung zuzuführen.

## Patentansprüche

1. Raspel (1), insbesondere zum einmaligen Gebrauch bestimmte Knochenraspel, **dadurch gekennzeichnet, dass** die Raspel einen die Aussenform der Raspel bestimmenden Stützkörper (3, 3') und eine den Stützkörper umgebende Umhüllung (4) aus Metall aufweist, welche die Raspelzähne (5) bildet.

2. Raspel nach Anspruch 1, **dadurch gekennzeichnet, dass** der Stützkörper (3) eine langgestreckte, sich zum hinteren Ende hin verbreiternde Form aufweist.

3. Raspel nach Anspruch 1, **dadurch gekennzeichnet, dass** der Stützkörper (3') eine im wesentlichen halbkugelförmige Form aufweist.

4. Raspel nach einem der Ansprüche 1 bis 3, **dadurch gekennzeichnet, dass** der Stützkörper mehrere über die Raspelform verteilte Rippen (10, 15) aufweist, an welchen die Umhüllung am Stützkörper aufliegt.

5. Raspel nach Anspruch 4, **dadurch gekennzeichnet, dass** diese mehrere zwischen den Rippen gebildete Hohlräume (11-13, 16, 17) aufweist, welche mit Öffnungen der Raspelzähne in Verbindung stehen.

6. Raspel nach einem der Ansprüche 1 bis 5, **dadurch gekennzeichnet, dass** der Stützkörper einen Anschluss zur Verbindung desselben mit einer eine oszillierende oder einer eine rotierende Bewegung erzeugende Bearbeitungsmaschine oder mit einem Anschluss für ein Adapterstück (2) zur Verbindung mit einer solchen Bearbeitungsmaschine aufweist.

7. Raspel nach einem der Ansprüche 1 bis 6, **dadurch gekennzeichnet, dass** der Stützkörper aus Aluminium oder Kunststoff gebildet ist.

8. Raspel nach einem der Ansprüche 1 bis 7, **dadurch gekennzeichnet, dass** die Umhüllung aus Metallblech, insbesondere Chromstahl, mit einer Dicke von 0,2 mm bis 4,0 mm, bevorzugterweise von 0,2 mm bis 1,0 mm und insbesondere von 0,2 mm bis 0,8 mm gebildet wird.

9. Verfahren zur Herstellung einer Raspel, insbesondere einer zum einmaligen Gebrauch bestimmten Knochenraspel, **dadurch gekennzeichnet, dass** ein die Form der Raspel bestimmender Stützkörper (3, 3') gebildet wird, dass eine einteilige oder mehrteilige, mit ihrer Innenform der Aussenform des Stützkörpers angepasste Hülle (4) aus Metall mit daran angeordneten Raspelzähnen (5) gebildet, und dass die Hülle am Stützkörper mindestens teilweise zur Auflage gebracht und an diesem befestigt wird.

10. Verfahren nach Anspruch 9, **dadurch gekennzeichnet, dass** die Hülle einteilig durch Tiefziehen hergestellt wird.

11. Verfahren nach Anspruch 9, **dadurch gekennzeichnet, dass** die Hülle mehrteilig hergestellt wird, und dass die Hüllenteile untereinander vor der Befestigung der Hülle am Stützkörper verbunden werden, oder dass die Hüllenteile unter Einbezug des Stützkörpers zu einer im wesentlichen geschlossenen Hülle verbunden werden.

## Claims

1. A rasp (1), in particular useful as a one-time use bone rasp, **characterized in that** the rasp has a support unit (3, 3') having an outer shape determining an outer form shape of the rasp, and an envelope (4) of metal material surrounding said inner support unit, which envelope forms the rasp teeth (5).

2. A rasp according to claim 1, **characterized in that** the support unit (3) has a longitudinally extended form that widens at its back end.

3. A rasp according to claim 1, **characterized in that** the support unit (3') has an essentially hemispherical shape.

4. A rasp according to one of claims 1 to 3, **characterized in that** the support unit comprises several ribs (10, 15) distributed over the rasp, to which ribs the envelope is applied to the support unit.

5. A rasp according to claim 4, **characterized in that** it has several hollow spaces (11-13, 16, 17) formed between the ribs, which hollow spaces are joined with openings of the rasp teeth.

6. A rasp according to one of claims 1 to 5, **characterized in that** the support unit has a connection for joining the support unit with a treatment machine producing an oscillating or rotating motion, or with a connection for an adaptor piece (2) for joining with such a treatment machine.

7. A rasp according to one of claims 1 to 6, **characterized in that** the support unit is formed of aluminium or plastic.

8. A rasp according to one of claims 1 to 7, **characterized in that** the envelope is formed of sheet metal, in particular of chromium steel, with a thickness of 0.2 mm to 4.0 mm, preferably between 0.2 mm and 1,0 mm, and most preferably between 0.2 and 0.8 mm.

9. A process for the production of a rasp, in particular one useful as a one-time use bone rasp, **characterized in that** a support unit (3, 3') determining the form of the rasp is provided, that a one-part or multi-part envelope (4) is formed of metal material and has a plurality of rasp teeth disposed thereon, the envelope having an inner form conforming to the outer form of the support unit, and that the envelope is placed at least partially over said support unit and attached to the support unit.

10. A process according to claim 9, **characterized in that** the envelope is provided in one part by deep-drawing.

11. A process according to claim 9, **characterized in that** the envelope is provided as a multi-part envelope, and that the envelope parts are joined together prior to attaching said envelope to said support unit, or that the envelope parts and the support unit are joined to form an essentially closed envelope.

## Revendications

1. Râpe (1), en particulier une râpe à os à usage unique, **caractérisée en ce que** la râpe comporte un corps de support (3, 3'), définissant la forme extérieure de la râpe, et une enveloppe (4) en métal, qui entoure le corps de support et forme les dents de râpage (5).

2. Râpe selon la revendication 1, **caractérisée en ce que** le corps de support (3) a une forme allongée, s'élargissant vers l'extrémité postérieure.

3. Râpe selon la revendication 1, **caractérisée en ce que** le corps de support (3') a une forme sensiblement semi-sphérique.

4. Râpe selon l'une quelconque des revendications 1 à 3, **caractérisée en ce que** le corps de support comporte plusieurs nervures (10, 15) réparties sur la forme de la râpe et au niveau desquels l'enveloppe est en appui sur le corps de support.

5. Râpe selon la revendication 4, **caractérisée en ce que** ladite râpe comporte plusieurs cavités (11 à 13, 16, 17) formées entre les nervures, lesquelles sont reliées aux ouvertures des dents de râpage.

6. Râpe selon l'une quelconque des revendications 1 à 5, **caractérisée en ce que** le corps de support comporte un raccord par lequel ledit corps de support peut être relié à une machine de travail générant un mouvement oscillant ou un mouvement rotatif ou comporte un raccord pour une pièce d'adaptateur (2) permettant la liaison à une telle machine de travail.

7. Râpe selon l'une quelconque des revendications 1 à 6, **caractérisée en ce que** le corps de support est réalisé en aluminium ou en matière plastique.

8. Râpe selon l'une quelconque des revendications 1 à 7, **caractérisée en ce que** l'enveloppe est réalisée en tôle métallique, en particulier en acier au chrome, avec une épaisseur de 0,2 mm à 4,0 mm, de préférence de 0,2 mm à 1,0 mm et en particulier de 0,2 mm à 0,8 mm.

9. Procédé de réalisation d'une râpe, en particulier une râpe à os à usage unique, **caractérisé en ce qu'**il est réalisé un corps de support (3, 3') définissant la forme de la râpe, **en ce qu'**il est réalisé une enveloppe (4) en métal avec des dents de râpage (5) agencées sur cette dernière, laquelle est conçue en une ou plusieurs parties et dont la forme intérieure est adaptée à la forme extérieure du corps de support, et **en ce que** l'enveloppe, au moins en partie, est amenée en appui sur le corps de support et est fixée à celui-ci.

10. Procédé selon la revendication 9, **caractérisé en ce que** l'enveloppe est réalisée en une seule pièce par emboutissage profond.

11. Procédé selon la revendication 9, **caractérisé en ce que** l'enveloppe est réalisée en plusieurs parties et **en ce que** les parties de l'enveloppe sont reliées les unes aux autres avant la fixation de l'enveloppe sur le corps de support, ou **en ce que** les parties de l'enveloppe, conjointement avec le corps de support, sont reliées les unes aux autres pour former une enveloppe sensiblement fermée.
